(19) Europäisches Patentamt
European Patent Office
Office européen des brevets

(11) EP 0 910 469 B1

(12) # FASCICULE DE BREVET EUROPEEN

(45) Date de publication et mention de la délivrance du brevet:
**15.03.2000 Bulletin 2000/11**

(21) Numéro de dépôt: **97932889.5**

(22) Date de dépôt: **10.07.1997**

(51) Int Cl.[7]: **B01J 31/12**, B01J 37/02, C07C 6/10

(86) Numéro de dépôt international:
**PCT/FR97/01266**

(87) Numéro de publication internationale:
**WO 98/02244 (22.01.1998 Gazette 1998/03)**

(54) **PROCEDE DE METATHESE D'ALCANES**

VERFAHREN ZUR METATHESE VON ALKANEN

METHOD OF METATHESIS OF ALKANES

(84) Etats contractants désignés:
**AT BE CH DE DK ES FI FR GB GR IE IT LI LU MC NL PT SE**

(30) Priorité: **12.07.1996 FR 9609033**

(43) Date de publication de la demande:
**28.04.1999 Bulletin 1999/17**

(73) Titulaire: **CENTRE NATIONAL DE LA RECHERCHE SCIENTIFIQUE (CNRS)
75016 Paris (FR)**

(72) Inventeurs:
- **VIDAL, Véronique
F-69100 Villeurbanne (FR)**
- **THEOLIER, Albert
F-69150 Decines (FR)**
- **THIVOLLE-CAZAT, Jean
F-69270 Fontaines sur Saône (FR)**
- **BASSET, Jean-Marie
F-69100 Villeurbanne (FR)**

(74) Mandataire: **Colombet, Alain André et al
c/o Cabinet Lavoix,
2,Place d'Estienne d'Orves
75441 Paris Cedex 09 (FR)**

(56) Documents cités:
**EP-A- 0 076 371**
**WO-A-90/14323**
**US-A- 4 467 047**

EP 0 910 469 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a trait à un procédé de métathèse d'alcanes.

Il est bien connu que les alcanes communément appelés paraffines sont des molécules difficiles à transformer à cause de leur inertie chimique.

On sait transformer des alcanes par des réactions d'hydrogénolyse des liaisons carbone-carbone. Sur certains catalyseurs métalliques on a pu observer simultanément des réactions d'homologation d'alcanes, mais celles-ci restent toujours très minoritaires par rapport aux réactions d'hydrogénolyse. En effet, ces réactions sont toujours conduites en présence d'hydrogène, à des températures variant de 250 à 350°C, au contact de catalyseurs à base de métaux de transition sous forme massique, ou sous forme de films ou bien sous forme de particules métalliques supportées sur oxydes. Les meilleurs résultats semblent avoir été obtenus par Donohoe, Clarke et Rooney avec les alcanes linéaires de $C_5$ à $C_7$ sur film de tungstène (J. Chem. Soc. Farad. Trans I 1980. 76, 345 ; J. Chem. Soc. Chem. Commun. 1979, 648) ; la réaction s'avère plus lente d'un ordre de grandeur avec les alcanes ramifiés et inexistante avec les alcanes cycliques. Sarkany a également étudié l'homologation du butane et de divers alcanes en $C_4$ ou $C_5$ sur des catalyseurs de type noir de nickel, $Ni/SiO_2$, noir de platine, $Pt/SiO_2$ ou $Pd/Al_2O_3$ (J. Chem. Soc. Farad. Trans. I, 1986, 82, 103 ; J. Catal., 1984, 89, 14) ; cette réaction est encore favorisée dans le cas d'alcanes linéaires par rapport aux alcanes ramifiés mais demeure toujours un processus mineur à côté des réactions d'isomérisation (par exemple de butane en isobutane sur $Pt/SiO_2$) ou d'hydrogénolyse (sur $Ni/SiO_2$). L'homologation-aromatisation de pentane ou cyclopentane en benzène a été rapportée par Peter et Clarke sur film de rhodium allié avec cuivre, étain, or ou argent (J. Chem. Soc. Farad. Trans I, 1976, 72, 1201) ainsi que par Sarkany sur $Ni/SiO_2$ (J. Chem. Soc. Chem. Commun. 1980, 525) ; seuls les films de rhodium allié donnent une sélectivité en benzène convenable alors que l'hydrogénolyse reste prépondérante avec les catalyseurs au nickel, même pour des conversions réduites. Ainsi les performances de ces réactions connues d'homologation d'alcanes restent très modestes ou liées à un processus d'aromatisation.

Pourtant, si l'on savait transformer les alcanes en leurs homologues supérieurs, cela constituerait un moyen de valoriser certaines coupes pétrolières telles que notamment les coupes $C_4$ ou $C_5$. De nombreuses applications seraient envisageables dans le domaine des pétroles, des carburants, des polymères, de la chimie en synthèse organique, permettant d'allonger les chaînes latérales ou d'obtenir des hydrocarbures supérieurs branchés par d'autres routes que la catalyse acide ou superacide. On sait en effet que des indices d'octane élevés sont particulièrement recherchés dans le domaine des carburants. Il est aussi bien connu que les alcanes de bas poids moléculaire sont peu valorisables alors que les alcanes plus lourds ont un intérêt commercial plus grand.

La présente invention a donc pour objectif de fournir un procédé permettant de transformer les alcanes en leurs homologues supérieurs et inférieurs et ce avec une grande sélectivité.

Un autre objectif de l'invention est de fournir un tel procédé qui ait un large domaine d'application, pouvant recouvrir tout à la fois les domaines des pétroles, des carburants, des polymères, de la chimie en synthèse organique, l'allongement des chaînes latérales de composés en comportant, l'obtention d'hydrocarbures supérieurs branchés. Un tel objectif est donc notamment la valorisation d'alcanes légers en alcanes plus lourds plus intéressants industriellement.

Un autre objectif de l'invention est de fournir un tel procédé qui puisse être mis en oeuvre dans des conditions opératoires modérées, à savoir notamment à température et pression relativement basses.

Il a été découvert, et c'est l'objet de la présente invention, qu'en utilisant des catalyseurs, on peut réaliser la métathèse des alcanes en homologues supérieurs et inférieurs, avec une grande sélectivité et même à basse température, en particulier à des températures inférieures à 200°C.

L'invention a donc pour objet un procédé de métathèse d'alcanes de départ linéaires ou ramifiés, dans lequel l'on fait réagir le ou les alcanes de départ sur un catalyseur solide comprenant un hydrure métallique greffé et dispersé sur un oxyde solide. Il paraît probable que ce catalyseur joue le rôle de précurseur catalytique. Lorsqu'il est mis en présence des alcanes, ce catalyseur tend à former un complexe alkylmétal qui serait l'espèce catalytiquement active.

La réaction selon l'invention assure la métathèse des liaisons sigma-C-C et la transformation d'un alcane en ses homologues supérieurs et inférieurs. Ainsi, à partir d'un alcane, par exemple l'éthane, il est possible d'obtenir directement et successivement tous les alcanes supérieurs et en particulier des alcanes ramifiés.

La réaction peut s'écrire selon l'équation :

$$C_nH_{2n+2} \rightarrow C_{n-i}H_{2(n-i)+2} + C_{n+i}H_{2(n+i)+2} \qquad (I)$$

i = 1,2,3...n-1

L'invention permet ainsi la transformation directe d'alcanes légers en alcanes plus lourds et ainsi une valorisation de ces alcanes légers, en particulier des coupes pétrolières en $C_4$-$C_5$. Elle permet également l'allongement de

chaînes hydrocarbonées latérales sur certains composés cycliques.

De façon tout à fait préférée, les catalyseurs sont obtenus à partir d'un complexe organométallique de formule (II) suivante :

$$MR_a \quad (II)$$

où

- M est un métal de transition choisi parmi ceux des groupes 5 et 6 de la classification périodique des éléments, de préférence parmi tantale, tungstène et chrome;
- les groupements R sont des ligands hydrocarbonés identiques ou différents, saturés ou non, de préférence en $C_1$ à $C_{10}$, liés au M par un ou plusieurs carbones (les liaisons métal-carbone pouvant être des liaisons simples, doubles ou triples) ;
- a est inférieur ou égal à la valence de M qui est 5 ou 6.

Parmi les ligands appropriés, on peut notamment citer méthyle, néopentyle, néopentylidène, néopentylidyne, benzyle ou leurs mélanges, par exemple néopentyle-néopentylidène ou néopentyle-néopentylidine.

Les mélanges néopentyle-néopentylidène et néopentyle-néopentylidine sont particulièrement intéressants en complexe avec le tantale, respectivement le tungstène.

Selon un mode de réalisation particulièrement approprié à la mise en oeuvre du catalyseur solide, on effectue la dispersion et le greffage du composé organométallique sur un oxyde solide très anhydre. L'oxyde solide, par exemple la silice, est soumis à un traitement thermique poussé (dans le dessein d'assurer une déshydratation et une déshydroxylation), notamment entre 200 et 1100°C pendant plusieurs heures (par exemple de 10 à 20 heures). Bien entendu, l'homme de l'art prendra garde à ne pas dépasser la température de dégradation ou de limite de stabilité de l'oxyde solide qu'il a choisi d'utiliser. Pour la silice, on effectue la déshydratation entre 200 et 500°C, de préférence aux environs de 500°C, pour une réaction de simple déshydratation ou à une température supérieure à 500°C si l'on souhaite obtenir, en plus, la formation de ponts siloxanes en surface.

Pour transférer le complexe sur l'oxyde solide, on peut procéder notamment par sublimation ou en solution.

Dans le cas de la sublimation, le complexe organométallique à l'état solide est chauffé sous vide et dans des conditions de température assurant sa sublimation et sa migration à l'état de vapeur sur l'oxyde solide, qui lui se trouve préférentiellement à l'état pulvérulent ou sous forme de pastilles ou analogues. La sublimation est notamment réalisée entre 50 et 150°C, de préférence aux environs de 80°C. Le dépôt peut être contrôlé par exemple par spectroscopie infra-rouge.

Le greffage se fait par réaction du complexe avec les groupements fonctionnels du support (OH, Si-O-Si, etc.). Le greffage sera de préférence conduit à une température supérieure ou égale à la température ambiante.

Il peut être souhaitable d'éliminer l'excès de complexe n'ayant pas réagi et s'étant simplement adsorbé à la surface de l'oxyde par une sublimation inverse.

On effectue ensuite un traitement sous hydrogène ou en présence d'un autre réducteur approprié, dans des conditions conduisant à transformer les atomes de métal en hydrures par hydrogénolyse des ligands hydrocarbonés. On peut notamment travailler sous une pression comprise entre $10^{-2}$ et 100 bars, mais de préférence à la pression atmosphérique. En ce qui concerne la température, on peut travailler entre 25 et 400°C, plus généralement aux environs de 150°C. La réaction est conduite sur une durée suffisante, ce qui peut aller par exemple de 1 h à 24 h, notamment de 10 à 20 h, en particulier 15 h environ.

Dans la méthode générale qui vient d'être décrite, on peut remplacer la sublimation par une réaction en solution. Dans ce cas, le complexe organométallique est en solution dans un solvant organique classique, tel que benzène, toluène, pentane, éther, la condition étant d'être en milieu très anhydre. La réaction s'effectue par mise en suspension de l'oxyde solide, de préférence pulvérulent, dans cette solution de complexe métallique, ou encore par toute autre méthode assurant un contact adéquat entre les deux milieux. La réaction peut être conduite à la température ambiante et plus généralement entre 25 et 150°C.

Les catalyseurs présentent les caractéristiques remarquables suivantes :

- très bonne dispersion du métal sur l'oxyde solide, cette dispersion étant en grande partie, majoritairement ou totalement monoatomique s'agissant du métal ;
- la liaison entre l'atome métallique et l'oxygène de l'oxyde solide est très forte et permet de maintenir l'état de dispersion atteint ;
- le métal fixé sur le support est dans un état d'insaturation poussé ; sa couche électronique d présente un fort déficit

en électrons (moins de 16 électrons) ; dans les cas observés, on se situe aux environs de 10 électrons.

D'autres complexes précurseurs peuvent être utilisés dans la mesure où ils conduisent à un hydrure conforme, par la méthode décrite ou par toute autre voie de synthèse.

Les complexes métalliques sont supportés ou greffés et dispersés sur un oxyde solide ; parmi les supports de type oxyde, on peut citer de manière préférée la silice, l'alumine, les silices-alumines ou l'oxyde de niobium, les zéolithes, sans que la liste soit limitative.

Parmi ces catalyseurs, les hydrures de tantale, de tungstène ou de chrome greffés sur silice ou silice-alumine, sont plus particulièrement recommandés.

L'invention a donc pour objet l'utilisation de ces complexes métalliques dispersés et greffés sur oxyde solide pour la fabrication d'un catalyseur de métathèse d'alcanes.

Les alcanes linéaires convenant à la réalisation du procédé selon l'invention peuvent être choisis parmi les alcanes contenant au moins deux atomes de carbone ; le procédé pouvant s'appliquer aux alcanes jusqu'à $C_{30}$ et au delà ; des exemples préférés d'alcanes sont l'éthane, le propane, le butane, le pentane, etc.

Le procédé peut également s'appliquer aux alcanes ramifiés contenant quatre atomes de carbone ou plus, notamment jusqu'en $C_{30}$ ; des exemples préférés sont : l'isobutane, l'isopentane, le méthyl-2-pentane, le méthyl-3-pentane, le diméthyl-2,3-butane, etc.

Le procédé peut encore s'appliquer aux hydrocarbures cycliques (composés à un ou plusieurs cycles), par exemple cycles aromatiques ou cycles saturés, substitués par au moins une chaîne alcane linéaire ou ramifiée. A titre d'exemple, on peut citer les hydrocarbures cycliques, substitués avec au moins une chaîne alcane linéaire ou ramifiée, selon la formule générale (III) :

$$(CH_2)_x-CH-(CH_2)_y-CH_3 \qquad (III),$$

où :

x est supérieur ou égal à 2, de préférence compris entre 2 et 20 ;
y est supérieur ou égal à 0, de préférence compris entre 0 et 29.

Dans ces cas, la réaction agit sur les chaînes alcanes.

Les hydrocarbures selon l'invention, c'est-à-dire saturés linéaires, ramifiés et/ou cycliques substitués (dans ce cas la réaction portant sur les alcanes de substitution) peuvent être soumis à la réaction de métathèse de liaisons sigma-carbone-carbone, sur eux-même ou en présence d'un autre hydrocarbure.

Le procédé peut encore s'appliquer à des composés similaires comportant un ou plusieurs hétéroatomes tels que O, S, N.

Une première méthode d'application du procédé selon l'invention consiste à faire réagir l'hydrocarbure linéaire, ramifié ou cyclique substitué par réaction de métathèse sur lui même pour obtenir les alcanes homologues supérieurs et inférieurs.

Une deuxième méthode consiste à faire réagir entre eux au moins deux hydrocarbures choisis parmi les linéaires, ramifiés et cycliques substitués, de façon à obtenir les hydrocarbures ramifiés ou cycliques substitués, issus des réactions de métathèse de liaisons sigma-carbone-carbone. Plus simplement, on réalise la réaction de métathèse sur un mélange d'au moins deux hydrocarbures différents.

La réaction de métathèse des alcanes linéaires, ramifiés ou cycliques substitués est effectuée de préférence par passage de l'alcane en phase gaz sur le catalyseur solide ; la réaction en phase gaz peut être conduite à la pression atmosphérique ou au delà, mais à une pression inférieure ou égale à la pression de condensation de l'alcane isolé ou de l'alcane le plus lourd lorsqu'il y a plusieurs alcanes de départ. La réaction peut également s'effectuer en phase liquide dans l'alcane ou dans un mélange d'alcanes avec le catalyseur en suspension. La réaction peut également s'opérer en présence d'un gaz inerte tel que de préférence l'azote, l'hélium ou l'argon.

La réaction de métathèse selon l'invention peut être conduite en réacteur statique, c'est-à-dire avec une quantité fixe de réactifs introduite pour un cycle complet de réaction, en réacteur à recyclage, dans lequel on peut notamment recycler les alcanes obtenus, ou encore en réacteur dynamique, c'est-à-dire avec un passage, sur un lit de catalyseur, des flux de réactifs liquides ou gazeux.

La réaction de métathèse peut s'effectuer à des températures variant de 25 à 300°C, mais de préférence entre 100 et 200°C. La pression peut être comprise entre $10^{-2}$ et 100 bars. On préfère travailler à partir de la pression atmosphérique.

La réaction de métathèse d'un alcane donné conduit à la formation de ses alcanes supérieurs et inférieurs

comme le montre l'équation (I) avec une sélectivité élevée en alcane supérieur, le composé intéressant.

L'intérêt étant surtout de former les alcanes supérieurs, on peut prévoir un recyclage des alcanes obtenus lors de la réaction. Il peut s'agir aussi bien du recyclage de l'alcane inférieur que du recyclage de l'alcane supérieur obtenu afin de continuer la réaction vers l'obtention d'alcanes toujours supérieurs.

On peut éventuellement prévoir la séparation entre alcanes supérieurs et alcanes inférieurs, par exemple dans le dessein de recycler l'alcane inférieur ou l'alcane supérieur. On peut bien entendu également recycler l'ensemble, la réaction selon l'invention se faisant, de manière avantageuse, préférentiellement sur les alcanes inférieurs.

La réaction de métathèse selon l'invention trouve de nombreuses applications dans les domaines définis plus haut. L'une des applications principales concerne l'obtention d'un indice d'octane élevé pour les carburants afin d'améliorer la stabilité à la compression. Le procédé selon l'invention permet d'enrichir les carburants en alcanes lourds et/ou ramifiés, ce qui va dans le sens d'une augmentation de la stabilité à la compression.

L'invention va être maintenant décrite plus en détail à l'aide de modes de réalisation non limitatifs.

**Exemple 1 : préparation du catalyseur hydrure de tantale :**

La préparation du catalyseur hydrure de tantale de surface $[Ta]_s$-H peut être réalisée de la façon suivante : dans un réacteur en verre, le tris(néopentyl)néopentylidènetantale $Ta[-CH_2-CMe_3]_3[=CH-CMe_3]$ est sublimé à 80°C sur de la silice préalablement déshydroxylée à 500°C, de façon à greffer le complexe de tantale par une réaction à 25°C avec un ou plusieurs groupes hydroxyles de la surface de silice, réaction qui produit également du néopentane:

$$\equiv SiOH + Ta[-CH_2-CMe_3]_3[=CH-CMe_3] \rightarrow$$

$$\equiv SiO-Ta[-CH_2-CMe_3]_2[=CH-CMe_3] + (\equiv SiO)_2-Ta[-CH_2-CMe_3][=CH-CMe_3]+CMe4$$

Le mélange des complexes néopentyl-néopentylidène obtenus:

$$\equiv SiO-Ta[-CH_2-CMe_3]_2[=CH-CMe_3] \text{ et } (\equiv -SiO)_2-Ta[-CH_2-CMe_3][=CH-CMe_3]$$

est ensuite traité sous hydrogène à la pression atmosphérique, à 150°C, pendant 15h, de façon à former les espèces hydrures de tantale supportées ; cette réaction s'accompagne de l'hydrogénolyse des ligands néopentyles et néopentylidène produisant méthane, éthane, propane, isobutane et néopentane dans la phase gaz.

Une autre façon de préparer le catalyseur hydrure de tantale $[Ta]_s$-H est la suivante :

la silice est préalablement déshydroxylée à une température supérieure à 500°C (jusqu'à 1100°C) de façon à faire apparaître en surface des ponts siloxanes plus ou moins tendus issus de la condensation des groupes hydroxyles ; le tris(néopentyl) néopentylidène tantale $Ta[-CH_2-CMe_3]_3[=CH-CMe_3]$ est sublimé à 80°C et réagit aussi bien avec les groupes hydroxyles restant qu'avec les ponts siloxanes selon :

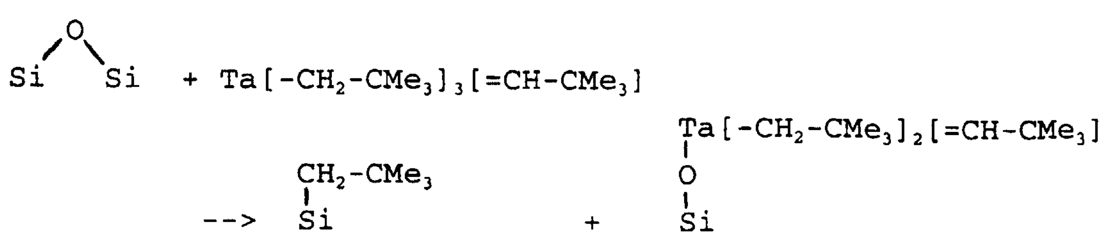

Le passage des complexes néopentyl-néopentylidène aux hydrures de tantale de surface se fait comme précédemment par traitement sous hydrogène.

**Exemple 2 : préparation du catalyseur hydrure de tungstène**

La préparation du catalyseur hydrure de tungstène de surface $[W]_s$-H peut être réalisée de la façon suivante : dans un réacteur en verre, le tris(néopentyl)néopentylidyne tungstène $W[-CH_2-CMe_3]_3[\equiv C-CMe_3]$ est sublimé à 80°C sur de la silice préalablement déshydroxylée à 500°C, de façon à greffer le complexe de tungstène par une réaction à 25°C avec un ou plusieurs groupes hydroxyles de la surface de silice. Le mélange des complexes du tungstène obtenus est ensuite traité sous hydrogène à la pression atmosphérique, à 150°C, pendant 15h, de façon à former les

espèces hydrures supportées ; cette réaction s'accompagne de l'hydrogénolyse des ligands néopentyl et néopentylidène produisant méthane, éthane, propane, isobutane et néopentane dans la phase gaz.

**Exemple 3 : réaction de métathèse de l'éthane :**

Le catalyseur hydrure de tantale $[Ta]_s$-H supporté sur la silice (52,2 mg ; 4,89 % Ta/$SiO_2$ ; 14,1 micromoles de Ta) est préparé in situ dans un réacteur en verre comme décrit ci-dessus. Le réacteur est mis sous vide, puis rempli d'éthane à pression atmosphérique (éthane/Ta = 800) et chauffé à 150°C en régime statique ; on observe alors la formation principalement de méthane et de propane ainsi que de butane et d'isobutane selon la tableau I :

tableau I :

| **Réaction de métathèse de l'éthane à 150°C.(éthane/Ta = 800)**. | | | | | |
|---|---|---|---|---|---|
| temps (h) | Rotations[a] | nombre de moles d'hydrocarbures formées par mole de tantale de surface | | | |
| | | $CH_4$ | $C_3H_8$ | isobutane | n-butane |
| 2,5 | 3,1 | 1,59 | 1,43 | 0,02 | 0,07 |
| 22 | 12,2 | 8,13 | 5,13 | 0,09 | 0,11 |
| 82 | 46,4 | 26,54 | 20,28 | 0,63 | 0,73 |

a : nombre de moles d'éthane transformées par rapport au tantale

**Exemple 4 : Réaction de métathèse du propane :**

Le catalyseur $[Ta]_s$-H (46,6 mg ; 4,44 % Ta/$SiO_2$ ; 11,4 micromole de Ta) est préparé comme dans l'exemple 3, puis le réacteur chargé de propane à pression atmosphérique (propane/Ta = 880) et chauffé à 150°C en statique. On obtient progressivement un mélange de méthane, d'éthane, de butane, d'isobutane et, en plus faible proportion, de pentane, d'isopentane et des homologues en $C_6$ selon le tableau 2 suivant.

**Exemple 5 : Réaction de métathèse du butane :**

Le catalyseur $[Ta]_s$-H (53,3 mg ; 4,78 % Ta/$SiO_2$ ; 14,1 micromole de Ta) est préparé comme dans l'exemple 3, puis le réacteur est chargé de butane à pression atmosphérique (butane/Ta = 900) et chauffé à 150°C en statique. On obtient progressivement un mélange d'éthane, de propane, de pentane et, en plus faibles proportions, de méthane, d'isobutane, d'isopentane et des homologues en $C_6$ selon le tableau 3 suivant.

**Exemple 6 : Réaction de métathèse de l'isobutane :**

Le catalyseur $[Ta]_s$-H (66 mg ; 9,52 % % Ta/$SiO_2$ ; 34,7 micromole de Ta) est préparé comme dans l'exemple 3, puis le réacteur est chargé d'isobutane à pression atmosphérique (isobutane/Ta = 244) et chauffé à 150°C en statique. On obtient progressivement un mélange de méthane, d'éthane, de propane, de néopentane, d'isopentane et de méthyl-2-pentane et en plus faibles proportions de n-butane et de méthyl-2-hexane selon le tableau 4 suivant.

**Exemple 7 : Réaction de métathèse du propane à 150°C sur catalyseur $[W]_s$-H/$SiO_2$ :**

Le catalyseur $[W]_s$-H (55,7 mg ; 4,96 % % W/$SiO_2$ ; 15,01 micromole de W) supporté sur silice est préparé comme décrit précédemment, puis le réacteur est chargé de propane à pression atmosphérique (propane/W = 790) et chauffé à 150°C en régime statique. On obtient progressivement un mélange de méthane, d'éthane, d'isobutane, de butane, de pentane et, en plus faible proportion, d'isopentane et d'hexane selon le tableau 5 suivant.

**Tableau 2 : Réaction de métathèse du propane à 150°C.(propane/Ta = 880).**

| temps (h) | Rotations | nombre de moles d'hydrocarbures formés par mole de tantale de surface | | | | | | | | |
|---|---|---|---|---|---|---|---|---|---|---|
| | | $CH_4$ | $C_2H_6$ | ibt[a] | nbut[b] | 2mbt[c] | npent[d] | C6[e] | C6[e] | nhex[f] |
| 5 | 14 | 3,32 | 5,95 | 1,05 | 4,34 | 0,34 | 0,70 | | | |
| 20 | 36,3 | 8,58 | 15,83 | 3,03 | 10,72 | 0,94 | 1,77 | | | |
| 43 | 43,8 | 9,39 | 18,07 | 3,58 | 12,91 | 1,13 | 2,22 | 0,11 | 0,09 | 0,32 |

a : i-butane, b : n-butane, c : 2-méthylbutane, d : n-pentane, e : isomères du n-hexane, f : n-hexane.

**Tableau 3 : Réaction de métathèse du butane à 150°C. (butane/Ta = 900).**

| temps (h) | Rotations | nombre de moles d'hydrocarbures formés par mole de tantale de surfac | | | | | | | | |
|---|---|---|---|---|---|---|---|---|---|---|
| | | $CH_4$ | $C_2H_6$ | $C_3H_8$ | ibt[a] | 2mbt[b] | npent[c] | C6[d] | C6[d] | nhex[e] |
| 1,25 | 7 | 0,58 | 1,06 | 3,83 | 1,4 | 0,24 | 1,40 | | | |
| 7,25 | 23,6 | 2,07 | 4,00 | 14,76 | 2,21 | 0,95 | 5,34 | | | |
| 23,25 | 40 | 2,38 | 6,24 | 22,71 | 2,55 | 1,15 | 7,86 | 0,32 | 1,16 | 3,13 |
| 138,25 | 66,5 | 2,78 | 10,66 | 36,33 | 3,00 | 1,97 | 14,29 | 0,58 | 0,32 | 5,72 |

a : i-butane, b : 2-méthylbutane, c : n-pentane, d : isomères du n-hexane, e : n-hexane.

**Tableau 4 : Réaction de métathèse de l'isobutane à 150°C. (isobutane/Ta = 244)**

| temps (h) | Rotations | Nombre de moles d'hydrocarbures formés par mole de tantale de surface | | | | | | | |
|---|---|---|---|---|---|---|---|---|---|
| | | $CH_4$ | $C_2H_6$ | $C_3H_8$ | $n\text{-}C_4$[a] | NpH[b] | $i\text{-}C_5$[c] | $i\text{-}C_6$[d] | $i\text{-}C_7$[e] |
| 3 | 3,3 | 1,38 | 0,87 | 1,33 | 0,15 | 0,3 | 0,44 | 0,27 | 0,04 |
| 23 | 9,5 | 2,23 | 3,28 | 2,38 | 0,08 | 0,4 | 1,79 | 1,53 | 0,23 |
| 47 | 11,5 | 2,71 | 4,96 | 2,95 | 0,09 | 0,65 | 2,02 | 1,62 | 0,15 |

a : n-butane, b : néopentane, c : isopentane, d : méthyl-2-pentane, e : méthyl-2-hexane.

**Tableau 5 : Réaction de métathèse du propane à 150°C sur catalyseur $[W]_s$-H/$SiO_2$ (propane/W = 790**

| temps (h) | Rotations | nombre de moles d'hydrocarbures formés par mole de tungstène de surface | | | | | | |
|---|---|---|---|---|---|---|---|---|
| | | $CH_4$ | $C_2H_6$ | ibt[a] | nbut[b] | 2mbt[c] | npent[d] | nhex[e] |
| 2 | 3,4 | 0,09 | 2,04 | 0,06 | 1,25 | | 0,14 | |
| 4 | 6,7 | 0,12 | 4,16 | 0,11 | 2,42 | | 0,33 | |
| 20 | 11,7 | 0,24 | 7,49 | 0,25 | 3,9 | 0,07 | 0,62 | 0,09 |

a : i-butane, b : n-butane, c : 2-méthylbutane, d : n-pentane, e : n-hexane.

## Revendications

1. Procédé de métathèse d'alcanes de départ linéaires ou ramifiés, dans lequel l'on fait réagir le ou les alcanes de départ sur un catalyseur solide comprenant un support formé d'un oxyde solide sur lequel sont greffés et dispersés des atomes métalliques sous forme d'hydrures.

2. Procédé selon la revendication 1, caractérisé en ce que les atomes métalliques sous forme d'hydrures du catalyseur présentent des degrés d'insaturation élevés.

3. Procédé selon la revendication 1 ou 2, caractérisé en ce que le catalyseur est tel qu'obtenu à partir

    a. d'un complexe organométallique de formule (II) suivante :

$$MR_a \quad \text{(II)}$$

    où

    - M est un métal de transition choisi parmi ceux des groupes 5 et 6 de la classification périodique des éléments ;
    - les groupements R sont de ligands hydrocarbonés identiques ou différents, saturés ou non, de préférence en $C_1$ à $C_{10}$, liés au M par un ou plusieurs carbones ;
    - a est inférieur ou égal à la valence de M qui est 5 ou 6;

    b. d'un oxyde solide anhydre.

4. Procédé selon la revendication 3, caractérisé en ce que l'oxyde solide anhydre est tel qu'obtenu par un traitement thermique pour déshydratation et déshydroxylation.

5. Procédé selon la revendication 3 ou 4, caractérisé en ce que le catalyseur est tel qu'obtenu par sublimation du complexe organométallique, puis son greffage sur l'oxyde solide, puis l'ensemble est soumis à une hydrogénolyse du ligand carboné afin de transformer le métal en hydrure.

6. Procédé selon la revendication 4 ou 5, caractérisé en ce que la sublimation est effectuée sous vide et entre 50 et 150°C, de préférence aux environs de 80°C.

7. Procédé selon la revendication 5 ou 6, caractérisé en ce que, la réaction de greffage est conduite à une température supérieure ou égale à la température ambiante.

8. Procédé selon la revendication 5, caractérisé en ce que, au lieu d'une sublimation, on effectue la dispersion et le greffage à partir d'une solution du complexe organométallique dans un solvant organique, et par mise en contact de cette solution avec l'oxyde solide.

9. Procédé selon l'une quelconque des revendications 3 à 8, caractérisé en ce que l'hydrogénolyse est conduite en présence d'hydrogène sous une pression comprise entre $10^{-2}$ et 100 bars, de préférence à la pression atmosphérique, et à une température comprise entre 25 et 400°C, de préférence aux environs de 150°C, pendant une durée allant de 1 h à 24 h.

10. Procédé selon l'une quelconque des revendications 1 à 9, caractérisé en ce que les atomes métalliques sont choisis parmi le groupe consistant en tantale, tungstène et chrome.

11. Procédé selon l'une quelconque des revendications 1 à 10, caractérisé en ce que l'oxyde solide est choisi parmi le groupe formé de silice, alumine, silice-alumine, oxyde de niobium, zéolithes.

12. Procédé selon la revendication 11, caractérisé en ce que le catalyseur est un hydrure de tantale, de tungstène ou de chrome greffé sur silice ou silice-alumine.

**13.** Procédé selon l'une quelconque des revendications 1 à 12, caractérisé en ce que la réaction de métathèse est conduite à une température comprise entre 25 et 300°C, de préférence entre 100 et 200°C.

**14.** Procédé selon l'une quelconque des revendications 1 à 13, caractérisé en ce que la réaction de métathèse est conduite par passage de l'alcane ou des alcanes en phase gazeuse sur le catalyseur solide.

**15.** Procédé selon la revendication 14, caractérisé en ce que la réaction de métathèse est conduite par passage de l'alcane ou des alcanes en phase gaz à une pression supérieure ou égale à la pression atmosphérique mais inférieure ou égale à la pression de condensation de l'alcane ou de l'alcane le plus lourd lorsqu'il y a plusieurs alcanes de départ.

**16.** Procédé selon l'une quelconque des revendications 1 à 15, caractérisé en ce que la réaction est mise en oeuvre sous une pression comprise entre $10^{-2}$ et 100 bars, de préférence à partir de la pression atmosphérique.

**17.** Procédé selon l'une quelconque des revendications 1 à 16, caractérisé en ce que la réaction de métathèse est conduite avec un catalyseur en suspension dans une phase liquide de l'alcane ou des alcanes.

**18.** Procédé selon l'une quelconque des revendications 1 à 17, caractérisé en ce que la réaction de métathèse est conduite en présence d'au moins un gaz inerte, de préférence choisi parmi le groupe consistant en azote, hélium, argon.

**19.** Procédé selon l'une quelconque des revendications 1 à 18, caractérisé en ce que l'alcane ou les alcanes de départ est/sont choisi(s) parmi le groupe consistant en les alcanes linéaires en $C_2$-$C_{30}$, les alcanes ramifiés en $C_4$-$C_{30}$ et les hydrocarbures cycliques, par exemple cycles aromatiques ou cycles saturés, substitués par au moins une chaîne alcane linéaire ou ramifiée.

**20.** Procédé selon la revendication 19, caractérisé en ce que l'hydrocarbure est un cycle saturé substitué selon la formule (III) :

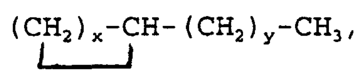

où :

x est supérieur ou égal à 2, de préférence compris entre 2 et 20
y est supérieur ou égal à 0, de préférence compris entre 0 e 29.

**21.** Procédé selon la revendication 19 ou 20, caractérisé en ce que le ou les alcanes est/sont choisi(s) parmi le groupe consistant en éthane, propane, butane, pentane, isobutane, isopentane, méthyl-2-pentane, méthyl-3-pentane, diméthyl-2,3-butane.

**22.** Procédé selon l'une quelconque des revendications 1 à 21 caractérisé en ce que l'on fait réagir ensemble au moins deux alcanes choisis parmi les alcanes linéaires, ramifiés et hydrocarbures cycliques substitués par au moins une chaîne linéaire ou ramifiée.

**Patentansprüche**

**1.** Verfahren zur Disproportionierung geradkettiger oder verzweigter Ausgangsalkane, in welchem das/die Ausgangsalkan/e an einem festen Katalysator umgesetzt wird/werden, der ein Trägermaterial enthält, das aus einem festen Oxid gebildet ist, auf welchem in Form von Hydriden vorliegende Metallatome gebunden und verteilt sind.

**2.** Verfahren nach Anspruch 1, **dadurch gekennzeichnet, daß** die in Form von Hydriden vorliegenden Metallatome des Katalysators einen hohen Ungesättigtheitsgrad aufweisen.

**3.** Verfahren nach Anspruch 1 oder 2, **dadurch gekennzeichnet, daß** der Katalysator ausgehend von

a. einem metallorganischen Komplex mit der Formel

$$MR_a \quad (II),$$

worin

- M ein aus den Übergangsmetallen der Gruppen 5 und 6 des Periodensystems der Elemente ausgewähltes Übergangsmetall bedeutet,
- die Reste R gegebenenfalls gesättigte gleiche oder verschiedene Liganden aus vorzugsweise $C_1$- bis
- $C_{10}$-Kohlenwasserstoffen, die über ein oder mehrere Kohlenstoffatome an M gebunden sind, bedeuten, und
- a kleiner oder gleich der Wertigkeit von M ist, die 5 oder 6 beträgt, und

b. einem wasserfreien festen Oxid

erhalten wird.

**4.** Verfahren nach Anspruch 3, **dadurch gekennzeichnet, daß** das wasserfreie feste Oxid derart ist, daß es durch eine Wärmebehandlung zur Dehydratation und Dehydroxylierung erhalten worden ist.

**5.** Verfahren nach Anspruch 3 oder 4, **dadurch gekennzeichnet, daß** der Katalysator derart ist, daß er durch Sublimation des metallorganischen Komplexes, dessen Verbindung mit dem festen Oxid und, indem das Ganze anschließend einer Hydrogenolyse des Kohlenstoff enthaltenden Liganden unterworfen wird, um das Metall in ein Hydrid umzuwandeln, erhalten worden ist.

**6.** Verfahren nach Anspruch 4 oder 5, **dadurch gekennzeichnet, daß** die Sublimation unter Vakuum und zwischen 50 und 150 °C und vorzugsweise bei etwa 80 °C durchgeführt wird.

**7.** Verfahren nach Anspruch 5 oder 6, **dadurch gekennzeichnet, daß** die Verbindungsreaktion bei einer Temperatur von oberhalb oder gleich der Umgebungstemperatur durchgeführt wird.

**8.** Verfahren nach Anspruch 5, **dadurch gekennzeichnet, daß** anstelle einer Sublimation die Verteilung und das Verbinden ausgehend von einer Lösung des metallorganischen Komplexes in einem organischen Lösungsmittel und durch In-Berührung-Bringen dieser Lösung mit dem festen Oxid durchgeführt wird.

**9.** Verfahren nach einem der Ansprüche 3 bis 8, **dadurch gekennzeichnet, daß** die Hydrogenolyse in Gegenwart von Wasserstoff bei einem Druck von $10^{-2}$ bis 100 bar und vorzugsweise bei Atmosphärendruck und einer Temperatur von 25 bis 400 °C und vorzugsweise etwa 150 °C in einem Zeitraum von 1 bis 24 h durchgeführt wird.

**10.** Verfahren nach einem der Ansprüche 1 bis 9, **dadurch gekennzeichnet, daß** die Metallatome aus der aus Tantal, Wolfram und Chrom bestehenden Gruppe ausgewählt sind.

**11.** Verfahren nach einem der Ansprüche 1 bis 10, **dadurch gekennzeichnet, daß** das feste Oxid aus der aus Siliciumdioxid, Aluminiumoxid, Siliciumdioxid-Aluminiumoxid, Nioboxid und den Zeolithen gebildeten Gruppe ausgewählt ist.

**12.** Verfahren nach Anspruch 11, **dadurch gekennzeichnet, daß** der Katalysator ein Tantal-, Wolfram- oder Chromhydrid ist, das mit Siliciumdioxid oder Siliciumdioxid-Aluminiumoxid verbunden ist.

**13.** Verfahren nach einem der Ansprüche 1 bis 12, **dadurch gekennzeichnet, daß** die Disproportionierungsreaktion bei einer Temperatur von 25 bis 300°C und vorzugsweise zwischen 100 und 200°C durchgeführt wird.

**14.** Verfahren nach einem der Ansprüche 1 bis 13, **dadurch gekennzeichnet, daß** die Disproportionierungsreaktion durch Leiten des/der Alkans/Alkane in der Gasphase über den festen Katalysator durchgeführt wird.

**15.** Verfahren nach Anspruch 14, **dadurch gekennzeichnet, daß** die Disproportionierungsreaktion durch Leiten des/der Alkans/Alkane in der Gasphase bei einem Druck von über oder gleich dem Atmosphärendruck, aber unter oder gleich dem Kondensationsdruck des/der schwersten Alkans/Alkane, falls mehrere Ausgangsalkane vorhanden sind, durchgeführt wird.

**16.** Verfahren nach einem der Ansprüche 1 bis 15, **dadurch gekennzeichnet, daß** die Umsetzung unter einem Druck von $10^{-2}$ bis 100 bar und vorzugsweise ausgehend vom Atmosphärendruck durchgeführt wird.

**17.** Verfahren nach einem der Ansprüche 1 bis 16, **dadurch gekennzeichnet, daß** die Disproportionierungsreaktion mit einem Katalysator durchgeführt wird, der in einer Flüssigphase aus dem/den Alkan/en suspendiert ist.

**18.** Verfahren nach einem der Ansprüche 1 bis 17, **dadurch gekennzeichnet, daß** die Disproportionierungsreaktion in Gegenwart mindestens eines Inertgases durchgeführt wird, das vorzugsweise aus der aus Stickstoff, Helium und Argon bestehenden Gruppe ausgewählt ist.

**19.** Verfahren nach einem der Ansprüche 1 bis 18, **dadurch gekennzeichnet, daß** das/die Ausgangsalkan/e aus der Gruppe ausgewählt ist/sind, die aus geradkettigen $C_2$-bis $C_{30}$-Alkanen, verzweigten $C_4$- bis $C_{30}$-Alkanen und cyclischen Kohlenwasserstoffen, beispielsweise aromatischen oder gesättigten Cyclen, die durch mindestens eine geradkettige oder verzweigte Alkankette substituiert sind, besteht.

**20.** Verfahren nach Anspruch 19, **dadurch gekennzeichnet, daß** der Kohlenwasserstoff ein gesättigter Cyclus ist, der gemäß Formel (III)

$$(CH_2)_x\text{-}CH\text{-}(CH_2)_y\text{-}CH_3$$

substituiert ist, worin

x größer oder gleich 2 und vorzugsweise 2 bis 20 und
y größer oder gleich 0 und vorzugsweise 0 bis 29

beträgt.

**21.** Verfahren nach Anspruch 19 oder 20, **dadurch gekennzeichnet, daß** das/die Alkan/e aus der aus Ethan, Propan, Butan, Pentan, Isobutan, Isopentan, 2-Methylpentan, 3-Methylpentan und 2,3-Dimethylbutan bestehenden Gruppe ausgewählt ist/sind.

**22.** Verfahren nach einem der Ansprüche 1 bis 21, **dadurch gekennzeichnet, daß** mindestens zwei Alkane miteinander umgesetzt werden, die aus geradkettigen oder verzweigten Alkanen und durch mindestens eine geradkettige oder verzweigte Kette substituierten cyclischen Kohlenwasserstoffen ausgewählt sind.

## Claims

**1.** Process for the metathesis of straight-chain or branched starting alkanes, wherein the starting alkane or alkanes is or are reacted on a solid catalyst comprising a substrate formed by a solid oxide on which metal atoms in the form of hydrides are grafted and dispersed.

**2.** Process according to claim 1, characterised in that the metal atoms in the form of hydrides of the catalyst have high levels of unsaturation.

**3.** Process according to claim 1 or 2, characterised in that the catalyst is of a kind which is obtained from

a. an organometallic complex of the following formula (II) :

$$MR_a \qquad (II)$$

wherein

- M is a transition metal selected from among those in groups 5 and 6 of the Period Table of Elements;
- the groups R are identical or different hydrocarbon ligands which may be saturated or unsaturated, preferably $C_1$ to $C_{10}$, linked to M by one or more carbons;
- a is less than or equal to the valency of M which is 5 or 6;

b. an anhydrous solid oxide.

**4.** Process according to claim 3, characterised in that the anhydrous solid oxide is of a kind which is obtained by heat treatment for dehydration and dehydroxylation.

**5.** Process according to claim 3 or 4, characterised in that the catalyst is of a kind obtained by sublimating the organometallic complex and then grafting it on to the solid oxide, after which the whole is subjected to hydrogenolysis of the carbon ligand in order to convert the metal into a hydride.

**6.** Process according to claim 4 or 5, characterised in that the sublimation is carried out *in vacuo* and between 50 and 150°C, preferably around 80°C.

**7.** Process according to claim 5 or 6, characterised in that the grafting reaction is carried out at a temperature above or equal to ambient temperature.

**8.** Process according to claim 5, characterised in that, instead of sublimation, the dispersion and grafting are carried out starting from a solution of the organometallic complex in an organic solvent and by bringing this solution into contact with the solid oxide.

**9.** Process according to any one of claims 3 to 8, characterised in that the hydrogenolysis is carried out in the presence of hydrogen under a pressure of between $10^{-2}$ and 100 bars, preferably at atmospheric pressure, and at a temperature of between 25 and 400°C, preferably about 150°C, for a period ranging from 1 h to 24 h.

**10.** Process according to any one of claims 1 to 9, characterised in that the metal atoms are selected from the group comprising tantalum, tungsten and chromium.

**11.** Process according to any one of claims 1 to 10, characterised in that the solid oxide is selected from the group comprising silica, alumina, silica-alumina, niobium oxide and zeolites.

**12.** Process according to claim 11, characterised in that the catalyst is a tantalum, tungsten or chromium hydride grafted onto silica or silica-alumina.

**13.** Process according to any one of claims 1 to 12, characterised in that the reaction of metathesis is carried out at a temperature of between 25 and 300°C, preferably between 100 and 200°C.

**14.** Process according to any one of claims 1 to 13, characterised in that the reaction of metathesis is carried out by passing the alkane or alkanes in gaseous phase over the solid catalyst.

**15.** Process according to claim 14, characterised in that the reaction of metathesis is carried out by passing the alkane or alkanes in gaseous phase at a pressure greater than or equal to atmospheric pressure but lower than or equal to the condensation pressure of the alkane or of the heaviest alkane when there are a plurality of starting alkanes.

**16.** Process according to any one of claims 1 to 15, characterised in that the reaction is carried out under a pressure of between $10^{-2}$ and 100 bars, preferably starting from atmospheric pressure.

**17.** Process according to any one of claims 1 to 16, characterised in that the reaction of metathesis is carried out with a catalyst suspended in a liquid phase of the alkane or alkanes.

**18.** Process according to any one of claims 1 to 17, characterised in that the reaction of metathesis is carried out in the presence of at least one inert gas, preferably selected from the group comprising nitrogen, helium and argon.

**19.** Process according to any one of claims 1 to 18, characterised in that the starting alkane or alkanes is or are selected from the group comprising straight-chain $C_{2-30}$ alkanes, branched $C_{4-30}$ alkanes and cyclic hydrocarbons, e.g. aromatic cyclic groups or saturated cyclic groups, which may be substituted by at least one straight or branched alkane chain.

**20.** Process according to claim 19, characterised in that the hydrocarbon is a substituted saturated cyclic group corresponding to formula (III):

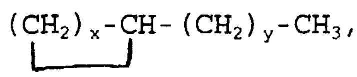

wherein:

x is greater than or equal to 2, preferably between 2 and 20,
y is greater than or equal to 0, preferably between 0 and 29.

**21.** Process according to claim 19 or 20, characterised in that the alkane or alkanes is/are selected from the group comprising ethane, propane, butane, pentane, isobutane, isopentane, methyl-2-pentane, methyl-3-pentane, dimethyl-2,3-butane.

**22.** Process according to any one of claims 1 to 21, characterised in that at least two alkanes selected from straight-chain alkanes, branched alkanes and cyclic hydrocarbons substituted by at least one straight or branched chain, are reacted together.